(19) Europäisches Patentamt
European Patent Office
Office européen des brevets

(11) Numéro de publication: **0 476 119 B1**

# (12) FASCICULE DE BREVET EUROPEEN

(45) Date de publication du fascicule du brevet: **27.12.95**

(51) Int. Cl.[6]: **B01J 29/08**, C10G 11/05

(21) Numéro de dépôt: **91907704.0**

(22) Date de dépôt: **08.04.91**

(86) Numéro de dépôt internationale :
**PCT/FR91/00287**

(87) Numéro de publication internationale :
**WO 91/15293 (17.10.91 91/24)**

## (54) CATALYSEUR A BASE D'UNE FAUJASITE ET SON APPLICATION

(30) Priorité: **09.04.90 FR 9004502**

(43) Date de publication de la demande:
**25.03.92 Bulletin 92/13**

(45) Mention de la délivrance du brevet:
**27.12.95 Bulletin 95/52**

(84) Etats contractants désignés:
**AT BE CH DE DK ES FR GB GR IT LI LU NL SE**

(56) Documents cités:
**EP-A- 0 194 101**
**EP-A- 0 247 678**
**EP-A- 0 364 352**
**EP-A- 0 366 207**
**FR-A- 2 066 550**
**FR-A- 2 093 794**
**GB-A- 1 536 590**
**US-A- 4 192 778**
**US-A- 4 493 902**
**US-H- 196**

**J.A. Rabo: "Zeolite chemistry and catalysis", 1976, American Chemical Society, (Washington, D.C., US), (ACS monograph 171) voir pages 35-46**

(73) Titulaire: **ELF AQUITAINE**
**Tour Elf,**
**2, Place de la Coupole,**
**La Défense 6**
**F-92400 Courbevoie (FR)**

(72) Inventeur: **DES COURIERES, Thierry**
**152, bd Yves-Farge**
**F-69007 Lyon (FR)**
Inventeur: **GUTH, Jean-Louis**
**59, rue Bellevue**
**F-68200 Brunstatt (FR)**
Inventeur: **PATARIN, Joel**
**13, rue Eugène-Delacroix**
**F-68093 Mulhouse (FR)**
Inventeur: **ZIVKOV, Catherine**
**Résidence l'Arrieulat,**
**13, rue du Gave**
**F-64300 Orthez (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

**Molecular Sieves (Conference School of Pharmacy, Londres), 1968, Society of Chemical Industry, (Londres, GB), W.M. Meier: "Zeolite structures", voir page**

(74) Mandataire: **Boillot, Marc**
**ELF AQUITAINE**
**Division Propriété Industrielle**
**Tour Elf**
**F-92078 Paris la Défense Cédex 45 (FR)**

## Description

L'invention concerne un catalyseur à base d'une zéolithe du type faujasite présentant un haut rapport Si:Al de synthèse. Elle se rapporte encore à l'application dudit catalyseur aux reactions de catalyse acide réalisées sur des charges hydrocarbonées.

Les zéolithes ont de nombreuses applications industrielles. Elles sont utilisées en particulier dans le domaine des adsorbants, des lessives ou de la catalyse industrielle. Dans ce dernier domaine, l'essentiel des applications telles que le craquage catalytique des distillats sous vide ou des résidus en essence ou encore l'hydrocraquage des distillats sous vide en gazole est satisfait par des catalyseurs renfermant une zéolithe de type faujasite dénommée zéolithe Y et possédant un rapport Si:Al de synthèse compris entre 1,5 et 3.

Ainsi la citation US-A-4493902 décrit un catalyseur de craquage en lit fluide de charges pétrolières, qui se présente sous la forme de microsphères renfermant au moins 40 % en poids d'une faujasite Y ayant un rapport molaire $SiO_2$:$Al_2O_3$ de synthèse d'environ 4,1 à 5,2 , ce qui correspond à un rapport Si:Al de synthèse d'environ 2,05 à 2,6 , et présentant un paramètre de sa maille cubique allant d'environ 2,464 à 2,473 nm.

La stabilité thermique et hydrothermique de la zéolithe Y et par conséquent des catalyseurs qui en contiennent reste faible et il est nécessaire de soumettre ladite zéolithe à des traitements post-synthèse pour améliorer ladite stabilité et plus généralement les performances desdits catalyseurs. Pour obtenir une augmentation de la stabilité thermique et hydrothermique de ladite zéolithe, il convient d'augmenter son rapport Si:Al de réseau et dans ce but on peut faire appel à divers traitements parmi lesquels on peut mentionner les techniques telles que traitement à la vapeur d'eau suivi ou non d'un lavage à l'acide des débris aluminiques (US-A-3493519 ; US-A-3506400), traitement par l'acide éthylène diamine tétracétique (G.T.KERR, J. Phys. Chem., 72 (1968), p.2594), traitement par $SiCl_4$ (H.K. BEYER, I.M BELENYKAJA, F. HANGE, M. TIELEN, O.J. GROBET et P.A. JACOBS, J. Chem. Soc., Farad. Trans.1, 81 (1985), pages 2889 à 2901) ou encore traitement au moyen de fluosilicate d'ammonium $(NH_4)_2SiF_6$ (US-A-4503023).

Tous ces traitements sont onéreux, ce qui conduit à une augmentation du coût du catalyseur final. De plus, de tels traitements engendrent des modifications structurales des zéolithes traitées avec, comme conséquence, l'apparition de défauts pouvant pénaliser les performances des catalyseurs incluant ces zéolithes traitées.

La citation EP-A-0247678 décrit, dans son exemple III, la préparation d'un catalyseur d'hydrocraquage de charges hydrocarbonées, ledit catalyseur incluant une zéolithe Y modifiée ayant un paramètre de maille cubique égal à 2,432 nm et un rapport molaire $SiO_2$:$Al_2O_3$ de 10,2, ce qui correspond à un rapport Si:Al de 5,1. La zéolithe Y modifiée résulte d'un traitement particulier de modification appliqué à une zéolithe Y ultra-stable commerciale ayant une teneur en $Na_2O$ de 0,12 % en poids et un rapport Si:Al d'environ 3 (rapport molaire $SiO_2$:$Al_2O_3$ d'environ 6), ledit traitement de modification comportant deux traitements d'échange d'ions avec une solution de sulfate d'aluminium, séparés par une calcination à 700 °C en présence de vapeur d'eau et suivis par un traitement final avec une solution de nitrate d'ammonium.

On a trouvé que des zéolithes du type faujasite ayant un rapport Si:Al de synthèse supérieur à 3 pouvaient être à la base d'excellents catalyseurs pour les réactions de catalyse acide de charges hydrocarbonées, en particulier réaction de craquage, d'hydrocraquage, d'isomérisation ou encore de dismutation, ces zéolithes présentant, entre autres, une densité élevée de sites acides de force moyenne ou grande et une excellente stabilité thermique et hydrothermique et pouvant également subir, avec beaucoup moins d'inconvénients que la zéolithe Y du fait de leur rapport Si:Al de réseau plus élevé, les traitements postsynthèse éventuels destinés, si besoin est, à augmenter ledit rapport.

L'invention propose donc un catalyseur comportant, en poids, y % d'un liant inorganique et z % d'une zéolithe protonée à structure faujasite de rapport Si:Al au moins égal à 3, y et z étant des nombres tels que $1 \leqq y \leqq 99$ et $1 \leqq z \leqq 99$ avec $y + z = 100$, ladite zéolithe présentant un paramètre $a_o$ de sa maille cubique tel que 2,42 nm $\leqq a_o \leqq$ 2,48 nm et répondant à une formule qui, ramenée à une maille cubique, s'écrit

$$(uM_1q+)\ (vH+)\ [(SiO_2)_{192-x}(AlO_2)_x]^{x-}\ (tH_2O)$$

avec dans cette formule x désignant un nombre tel que $6 \leqq x \leqq 48$, $M_1q+$ désignant des cations d'au moins un métal de valence q choisi parmi les métaux alcalins et les métaux alcalinoterreux, et u, v, x et t représentant des nombres tels que $t \geqq 0$ selon le degré d'hydratation de la zéolithe, $t = 0$ pour une zéolithe complètement anhydre, et que $qu + v \geqq x$, ledit catalyseur se caractérisant en ce que le nombre v de cations $H^+$ dans la formule de la zéolithe protonée est tel que $0{,}5x \leqq v \leqq x$ et de préférence $0{,}8x \leqq v \leqq x$ et en ce que ladite zéolithe protonée est issue d'une faujasite alcaline de rapport Si:Al de synthèse au moins

égal à 3 ou d'une telle faujasite désaluminée, laquelle faujasite alcaline est obtenue en formant un gel d'aluminosilicate à partir d'un mélange réactionnel, qui a un pH supérieur à 10 et renferme de l'eau, une source de silicium tétravalent, une source d'aluminium trivalent, une source d'ions hydroxydes sous la forme d'hydroxyde de métal alcalin ou alcalinoterreux et un agent structurant ST consistant en au moins un composé choisi dans le groupe formé (i) des macrocycles et macropolycycles comportant dans le cycle ou les cycles des hétéoatomes pris parmi O, N, Si et S et renfermant 10 à 17 atomes dans le cycle pour les macrocycles et 10 à 18 atomes dans chaque cycle pour les macropolycycles et (ii) des composés acycliques oxygénés de formule

$$R_1O-\left[C_mH_{2m-1}\ Y-O\right]_g-R_2,$$

dans laquelle R1 et R2, identiques ou différents, représentent chacun un atome d'hydrogène ou un radical alcoyle en C1 à C4, Y désigne un atome d'hydrogène ou un radical -OH, m est égal à 2 ou 3 et peut être différent d'un motif à l'autre et g est un nombre allant de 1 à 12, ledit gel d'aluminosilicate présentant, en termes de proportions molaires, une composition telle que l'on ait 1 $Al^{III}$ : 2 à 20 $Si^{IV}$ : 0,5 à 8 $OH^-$ : 0,1 à 4 ST : 40 à 200 $H_2O$, puis en maintenant le gel obtenu à une température au plus égale à 150 °C et sous une pression au moins égale à la pression autogène du mélange constitué par ledit gel pendant une durée suffisante pour effectuer la cristallisation de ce gel en un précurseur de la faujasite alcaline consistant en ladite faujasite emprisonnant l'agent structurant ST dans ses cavités et en soumettant ledit précurseur à une calcination pour détruire l'agent structurant et produire la faujasite alcaline.

Dans le catalyseur selon l'invention, les pourcentages pondéraux y de liant inorganique et z de zéolithe sont de préférence tels que $4 \leq y \leq 96$ et $4 \leq z \leq 96$ avec $y + z = 100$.

En plus du liant et de la zéolithe, le catalyseur peut encore renfermer, en poids de l'ensemble liant et zéolithe, jusqu'à 15 % et de préférence jusqu'à 10 % d'au moins un métal catalytiquement actif, présent à l'état élémentaire ou sous la forme d'un composé métallique et choisi parmi les métaux nobles tels que palladium, platine, rhodium et iridium, les métaux de transition non nobles tels que nickel, cobalt, molybdène et tungstène, et certains autres métaux tels que gallium.

Dans la formule de la zéolithe, $M_1q+$ représente en particulier des cations d'au moins un élément choisi parmi Na, K, Li, Cs, Ca, Sr et Ba.

La quantité d'agent structurant ST présente dans le mélange réactionnel destiné à former le gel d'aluminosilicate est telle que le rapport molaire ST:$Al^{III}$ aille de 0,1 à 4, ledit rapport allant de préférence de 0,1 à 1 et tout particulièrement de 0,2 à 0,5.

Comme indiqué plus haut, les ingrédients constituant le mélange réactionnel donnant naissance au gel d'aluminosilicate sont utilisés de telle sorte que ledit gel ait, en termes de rapports molaires, la composition suivante 1 $Al^{III}$ : 2 à 20 $Si^{IV}$ : 0,5 à 8 $OH^-$ : 0,1 à 4 ST : 40 à 200 $H_2O$. De préférence, ladite composition est telle que 1 $Al^{III}$ : 4 à 10 $Si^{IV}$ : 1 à 6 $OH^-$ : 0,1 à 1 ST : 50 à 150 $H_2O$. Ces conditions larges et préférées sont résumées dans le tableau suivant.

| | Intervalles larges | Intervalles préférés |
|---|---|---|
| $Si^{IV}$ : $Al^{III}$ | 2 à 20 | 4 à 10 |
| $OH^-$ : $Al^{III}$ | 0,5 à 8 | 1 à 6 |
| ST : $Al^{III}$ | 0,1 à 4 | 0,1 à 1 |
| $H_2O$ : $Al^{III}$ | 40 à 200 | 50 à 150 |

Les agents structurants ST utilisables pour constituer le mélange réactionnel donnant naissance au gel d'aluminosilicate peuvent être notamment

- des éthers couronnes dont le cycle renferme 10 à 17 atomes et comporte uniquement des atomes d'oxygène comme hétéroatomes, parmi lesquels on peut citer les composés suivants :
  - 1,4,7,10-tétraoxacyclododécane (éther couronne "12 crown 4"),
  - 1,4,7,10,13-pentaoxacyclopentadécane (éther couronne "15 crown 5"),
  - 2,3,-benzo 1,4,7,10,13,-pentaoxacyclopentadécane (éther couronne "benzo 15 crown 5"),
- des composés ayant une structure comparable à celle des éthers couronnes ci-dessus mais dont les atomes d'oxygène du cycle sont remplacés en partie ou en totalité par des substituants choisis parmi les atomes de soufre et les groupement $>$NH, $>$NR et

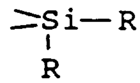

dans lesquels R est un radical hydrocarbyle en $C_1$ à $C_4$, parmi lesquels on peut citer les composés suivants :

1,4,8,11-tétrazacyclotétradécane ,

1,4,8,12-tétrazacyclopentadécane ,

1,4,8,11-tétrazacyclotridécane ,

14-(diméthyl-1,1 sila) 1,4,7,10,13-pentaoxacyclotétradécane (éther couronne "diméthylsila 14 crown 5"),

11-(diméthyl-1,1 sila) 1,4,7,10-tétraoxacycloundécane (éther couronne "diméthylsila 11 crown 4") et sont dérivé 3, 6,9-méthyl,

17-(diméthyl-1,1 sila) 1,4,7,10,13,16-hexaoxacycloheptadécane (éther couronne "diméthylsila 17 crown 6"),

17-(méthyl-1 vinyl-1 sila) 1,4,7,10,13,16-hexaoxacycloheptadécane (éther couronne "méthylvinylsila 17 crown 6"),

14-(méthyl-1 vinyl-1 sila) 1,4,7,10,13-pentaoxacyclo-tétradécane (éther couronne "méthylvinylsila 14 crown 5"),

1,7,10-trioxa 4,13-diazacyclopentadécane (Kryptofix 2.1);

- des macropolycycles carbonés du type des polyoxadiazabicycloalcanes dont chaque cycle renferme 10 à 18 atomes et possède au moins deux atomes d'oxygène en plus des deux atomes d'azote, parmi lesquels on peut citer les composés suivants :

  4,7,13,16,21-pentaoxa 1,10-diazabicyclo(8.8.5)-trico-sane (Kryptofix 2.2.1)

  4,7,13,16,21,24-hexaoxa 1,10-diazabicyclo(8.8.8)-hexacosane (Kryptofix 2.2.2) ;

- des composés acycliques oxygénés répondant à la formule générale précédente tels que méthyléther d'éthylèneglycol de formule $CH_3O\ CH_2\ CH_2OH$, diméthyléther d'éthylène glycol de formule $CH_3O\ CH_2\ CH_2\ OCH_3$, éthylèneglycol de formule $HO\ CH_2\ CH_2\ OH$, propylèneglycol de formule $HO\ CH_2\ CH_2\ CH_2\ OH$, éthers méthyliques de polyéthylèneglycols de formule

$$CH_3-O-\left[CH_2\ \ CH_2O\right]_{g_1}-H$$

et polyéthylèneglycols de formule

$$HO-\left[CH_2\ \ CH_2\ \ O\right]_{g_1}H$$

avec $g_1$ allant de 2 à 9 et notamment tétraéthylèneglycol, pentaéthylèneglycol, hexaéthylèneglycol, heptaéthylèneglycol, octaéthylèneglycol et mélanges de tels glycols, polypropylèneglycols de formule

$$HO-\left[CH_2\ \ CH_2\ \ CH_2\ \ O\right]_{g_1}-H$$

avec $g_1$ allant de 2 à 9 et notamment tripropylèneglycol, tétrapropylèneglycol.

Parmi les sources de silicium tétravalent $Si^{IV}$ utilisables dans la préparation du mélange réactionnel destiné à former le gel d'aluminosilicate, on peut citer les silices solides finement divisées sous la forme d'hydrogels, d'aérogels ou de suspensions colloidales, les silicates hydrosolubles tels que les silicates alcalins comme le silicate de sodium, les esters siliciques hydrolysables tels que les orthosilicates de tétraalcoyle de formule $Si(OR)_4$ dans laquelle R est un alcoyle en $C_1$ à $C_4$ tel que méthyle ou éthyle. La source de silicium est mise en oeuvre sous la forme d'une solution aqueuse vraie, cas des silicates hydrosolubles, ou bien d'une suspension aqueuse qui peut être colloidale, cas des silices finement divisées.

Conviennent comme sources d'aluminium trivalent $Al^{III}$ les sels d'aluminium tels que sulfate, nitrate, chlorure, fluorure, acétate, les oxydes et hydroxyoxydes d'aluminium, les aluminates et notamment les

aluminates alcalins tels que l'aluminate de sodium, les esters d'aluminium tels que les aluminium trialcoxydes de formule $Al(OR)_3$ dans laquelle R désigne un radical alcoyle en $C_1$ à $C_4$ tel que méthyle, éthyle ou propyle.

La source d'ions hydroxydes est choisie notamment parmi les hydroxydes des métaux alcalins, les hydroxydes des métaux alcalino-terreux Ca, Sr et Ba et les bases fortes organiques, notamment hydroxydes d'ammoniums quaternaires, la préférence allant aux bases minérales et notamment à la soude NaOH.

Le mélange des ingrédients constituant le milieu réactionnel destiné à former le gel d'aluminosilicate peut être réalisé dans un ordre quelconque. Avantageusement on effectue ledit mélange en préparant tout d'abord, à température ambiante, une solution aqueuse basique renfermant une base forte et l'agent structurant, puis en incorporant à cette solution une solution aqueuse de la source d'aluminium trivalent et une solution aqueuse ou une suspension, colloidale ou non, de la source de silicium tétravalent.

Le pH du mélange réactionnel, dont la valeur est supérieure à 10, est de préférence proche de 13.

Avant de procéder à la cristallisation du gel, on peut ajouter au milieu réactionnel destiné à former ledit gel, des germes de cristallisation en quantité allant avantageusement de 0, 1 à 10 % en poids du milieu réactionnel. Ces germes peuvent être produits soit par broyage d'une zéolithe de même nature que la phase cristalline à produire ou encore par synthèse à partir d'une solution de nucléation appropriée. Une telle solution a par exemple la composition suivante, exprimée en oxydes :

15 $Na_2O$ ; 1 $Al_2O_3$ ; 10 $SiO_2$ ; 180 $H_2O$.

En l'absence d'ajout de germes, il est avantageux de soumettre le gel d'aluminosilicate, formé à partir du mélange réactionnel, à un mûrissement, dans une enceinte fermée, à une température inférieure à la température de cristallisation pendant une durée pouvant aller d'environ 6 heures à environ 6 jours. Ledit mûrissement peut être réalisé en statique ou sous agitation.

La cristallisation du gel d'aluminosilicate, avec ou sans germe, s'effectue en chauffant le mélange réactionnel à une température au plus égale à 150 °C et de préférence allant de 90 °C à 120 °C et sous une pression correspondant au moins à la pression autogène du mélange réactionnel formant le gel. La durée du chauffage nécessaire à la cristallisation se situe généralement entre 2 heures et quinze jours.

Les cristaux obtenus, qui consistent en la zéolithe alcaline emprisonnant l'agent structurant dans ses pores et cavités, sont séparés du milieu de cristallisation par filtration, puis lavés à l'eau distillée ou désionisée jusqu'à obtenir des eaux de lavage dont le pH est inférieur à 9. Les cristaux lavés sont ensuite séchés en étuve à une température comprise entre 50 °C et 100 °C et de préférence aux environs de 70 °C.

La zéolithe alcaline est obtenue à partir des cristaux séchés en soumettant lesdits cristaux à une calcination à une température supérieure à 300 °C et de préférence comprise entre 400 °C et 700 °C pendant une durée suffisante pour éliminer l'agent structurant qu'ils renferment.

La forme protonée (acide) de la zéolithe est préparée, à partir de la forme alcaline obtenue comme décrit ci-dessus, en effectuant tout d'abord une mise en contact de la zéolithe alcaline calcinée avec une solution aqueuse d'un sel d'ammonium en opérant à des températures allant, par exemple, de 50 °C à 100 °C pour réaliser un échange des cations de métal alcalin ou alcalino-terreux de la zéolithe alcaline par des cations $NH_4^+$, l'opération étant répétée plusieurs fois si besoin est, puis en soumettant la zéolithe renfermant des cations $NH_4^+$ à l'action de températures appropriées pour effectuer une dégradation thermique des cations $NH_4^+$ et obtenir la forme acide de la zéolithe.

Le procédé de synthèse décrit plus haut conduit à des zéolithes dont le rapport Si:Al de réseau est égal ou supérieur à 3. Si besoin est, on peut encore augmenter ledit rapport Si:Al en soumettant la zéolithe, dans sa forme alcaline ou acide ou échangée, à l'un des traitements de désalumination connus à cet effet et, par exemple, à l'un des traitements à la vapeur d'eau, à l'acide éthylène-diaminetétracétique, par $SiCl_4$ ou par $(NH_4)_2SiF_6$ cités précédemment.

Comme indiqué plus haut, les zéolithes utilisées dans le catalyseur selon l'invention sont des faujasites, c'est-à-dire des zéolithes ayant une structure de symétrie cubique, qui ont un rapport Si:Al de réseau allant de 3 à 31 et de préférence de 3 à 20. Ces zéolithes ont un paramètre $a_o$ de leur maille cubique tel que 2,42nm $\leq a_o \leq$ 2,48nm et présentent, après calcination à 600 °C pendant 4 heures, un diagramme de diffraction des rayons X comparable à celui du tableau I ci-après.

Le rapport Si:Al de la zéolithe peut être déterminé par analyse chimique, ou bien par radiocristallographie (D.W.BRECK:"Zeolite Molecular Sieves", Ed. John Wiley and Sons, Mers York, 1974, page 94) ou encore par RMN du silicium 29 (J. KLINOWSKI : "Progress in NMR Spectroscopy", 1984, Vol.16, pages 237 à 309).

Le diagramme de diffraction des rayons X est obtenu au moyen d'un diffractomètre en utilisant la méthode classique des poudres avec le rayonnement $K_\alpha$ du cuivre. Un étalon interne permet de déterminer précisément les valeurs des angles $2\theta$ associés aux pics de diffraction. Les différentes distances interréti-

culaires $d_{hkl}$ , caractéristiques de l'échantillon, sont calculées à partir de la relation de BRAGG. L'intensité relative I:Io affectée à chaque valeur $d_{hkl}$ est estimée à partir de la hauteur du pic de diffraction correspondant. Pour caractériser cette intensité relative on utilise une échelle de symbole comme suit : FF = très forte, F = forte, mF = moyennement forte, m = moyenne, mf = moyennement faible, f = faible et ff = très faible.

Dans la colonne des $d_{hkl}$ du tableau I, on a donné les valeurs moyennes des distances interréticulaires. Chacune de ces valeurs doit être affectée de l'erreur de mesure $\Delta$ ($d_{hkl}$ ) comprise en ± 0,2 et ± 0,008. Les variations qui peuvent être observées par rapport à ces valeurs moyennes sont essentiellement liées à la nature des cations de compensation et au rapport Si:Al de la zéolithe. Les mêmes remarques s'appliquent aux intensités relatives I/Io.

TABLEAU I

| $2\theta$ (°) | $d_{hkl}$ ($10^{-1}$nm) | (h k l) | I/Io |
|---|---|---|---|
| 6,245 | 14,14 ± 0,2 | (1 1 1) | FF |
| 10,205 | 8,66 | (2 2 0) | F |
| 11,965 | 7,39 | (3 1 1) | mF |
| 15,735 | 5,627 ± 0,05 | (3 3 1) | F |
| 18,775 | 4,721 | (5 1 1) | f |
| 20,465 | 4,335 | (4 4 0) | mf |
| 22,895 | 3,881 | (6 2 0) | f |
| 23,755 | 3,727 | (5 3 3) | mF |
| 25,105 | 3,544 | (4 4 4) | ff |
| 25,965 | 3,428 | (5 5 1) | ff |
| 27,175 | 3,279 | (6 4 2) | mF |
| 27,885 | 3,196 ± 0,008 | (7 3 1) | ff |
| 29,765 | 2,999 | (7 3 3) | f |

Le liant inorganique que l'on associe à la zéolithe pour former le catalyseur selon l'invention peut être inerte, c'est-à-dire sans action catalytique dans la réaction pour laquelle le catalyseur est utilisé, ou bien être actif, c'est-à-dire présenter une certaine activité catalytique pour la réaction considérée. Le liant inorganique peut consister notamment en un ou plusieurs oxydes réfractaires comme, par exemple, alumine, silice, silice/alumine, argile, magnésie. Le liant inorganique peut également consister en un catalyseur présentant une activité pour une réaction et auquel l'addition d'une zéolithe selon l'invention améliore substantiellement ladite activité ou apporte une activité catalytique pour une autre réaction.

Le catalyseur selon l'invention peut être préparé à partir du liant inorganique et de la zéolithe en faisant appel aux diverses techniques proposées à cet effet. On peut en particulier faire appel à une technique d'extrusion consistant à préparer une pâte à partir de quantités appropriées du liant inorganique et de la zéolithe et d'une petite quantité d'eau, puis à soumettre la pâte ainsi formée à une extrusion à travers une filière pour former des granulés. On peut encore préparer le catalyseur sous la forme de microsphères par séchage par atomisation d'une suspension aqueuse du liant inorganique et de la zéolithe. On peut également mettre en forme le catalyseur à partir du liant inorganique et de la zéolithe en utilisant une technique de pastillage ou de dragéification.

La mise en forme du catalyseur peut être encore effectuée à partir du liant inorganique et de la forme alcaline de la zéolithe, après quoi le produit résultant de cette mise en forme est soumis aux opérations décrites précédemment pour obtenir la forme de la zéolithe renfermant des cations $H^+$.

Lorsque le catalyseur selon l'invention renferme un métal ou un composé métallique en plus des cations de compensation de la zéolithe, ledit métal ou composé métallique est incorporé au catalyseur par toute méthode connue, par exemple, par addition au mélange du liant inorganique et de la zéolithe avant la mise en forme dudit mélange, par imprégnation du liant inorganique et/ou de la zéolithe avant mélange de ces derniers ou encore par imprégnation du produit résultant de la mise en forme du mélange du liant inorganique et de la zéolithe.

Le catalyseur selon l'invention est utilisable pour promouvoir les réactions de catalyse acide réalisées sur des charges hydrocarbonées.

En particulier ledit catalyseur convient bien pour le craquage catalytique de charges hydrocarbonées, notamment craquage catalytique de distillats sous vide ou de résidus en essence, en opérant en particulier en lit fluidisé ("Fluid Catalytic Cracking" abrégé en "FCC") ou en lit mobile à des températures allant, par

exemple, de 450 °C à 650 °C.

Le catalyseur selon l'invention convient encore pour promouvoir l'hydrocraquage de charges hydrocarbonées, en particulier hydrocraquage de distillats sous vide en gazole, en opérant à des températures supérieures à 250 °C, notamment entre 350 °C et 500 °C, sous une pression supérieure à 15 bars, avantageusement supérieure à 30 bars et avec une vitesse volumique horaire comprise entre 0,2 et 10.

Le catalyseur précité convient également pour promouvoir l'alkylation des hydrocarbures aromatiques, notamment benzène, par les oléfines, ainsi que l'isomérisation des hydrocarbures alkylaromatiques tels que les xylènes.

La zéolithe présente dans le catalyseur possède une surface spécifique, déterminée par adsorption d'azote selon la méthode B.E.T. simplifiée à un point (norme NF X 11-622), comprise entre 100 $m^2/g$ et 1000 $m^2/g$ et de préférence entre 300 $m^2/g$ et 900 $m^2/g$.

Les exemples suivants sont donnés à titre non limitatif pour illustrer l'invention.

EXEMPLE 1 :

Synthèse d'une faujasite acide présentant un rapport Si:Al supérieur à 3.

On préparait tout d'abord un gel d'aluminosilicate en opérant comme suit dans un récipient de capacité appropriée, le contenu dudit récipient étant maintenu sous agitation pendant toute la durée de l'opération.

Dans le récipient on introduisait 9 parties d'eau, puis 0,44 partie de soude NaOH et, après dissolution de la soude, 1,22 partie d'éther couronne "15 crown 5". Après dissolution totale de l'éther couronne, on ajoutait alors au contenu du récipient 1 partie d'un aluminate de sodium renfermant 56% d'$Al_2O_3$ et 37% de $Na_2O$ et chauffait légèrement le mélange réactionnel pour dissoudre complètement l'aluminate. Après retour à température ambiante, on introduisait alors dans le récipient 8,2 parties d'une suspension colloidale de silice renfermant 40% de $SiO_2$ et 60% d'eau.

On obtenait ainsi un gel d'aluminosilicate dont la composition molaire, rapportée à une mole d'$Al_2O_3$, était la suivante :

$10SiO_2$ ; $1Al_2O_3$ ; 2,1 $Na_2O$ ; 1 éther couronne ; $140H_2O$

Le gel obtenu était soumis à un mûrissement à température ambiante pendant 24 heures dans un récipient fermé.

Le gel mûri était ensuite placé dans un autoclave et maintenu à 110 °C dans ce dernier pendant 336 heures pour former un produit cristallisé. Les cristaux formés étaient séparés du milieu réactionnel par filtration, puis lavés à l'eau distillée jusqu'à faible basicité (pH inférieur à 9) des eaux de lavage et enfin séchés à environ 60 °C dans une étuve.

Les cristaux séchés étaient ensuite calcinés afin d'éliminer l'éther couronne utilisé comme agent structurant, ladite calcination étant réalisée sous un léger débit d'air égal à 1,5 1/heure selon le profil thermique suivant

| rampe de température | | durée (mn) |
|---|---|---|
| 20 °C | 100 °C | 30 |
| 100 °C | 100 °C | 60 |
| 100 °C | 200 °C | 30 |
| 200 °C | 200 °C | 60 |
| 200 °C | 450 °C | 60 |
| 450 °C | 450 °C | 180 |
| 450 °C | 25 °C | (inertie du four) |

Le solide obtenu après calcination qui consiste en la forme alcaline de la zéolithe, présentait un diagramme de diffraction des rayons X comparable à celui du tableau I caractéristique des faujasites calcinées.

La formule trouvée pour cette zéolithe, ramenée à une maille de la structure cubique, s'écrit à l'état anhydre

$$38{,}5\ Na^+\ [(SiO_2)_{154,0}\ (AlO_2)_{38,0}]^{38,0-}$$

On observe un très léger excès de charge positive par rapport à la neutralité.

Le solide calciné, consistant en la forme sodique calcinée de la zéolithe, était mis en contact avec une solution deux fois molaire de $NH_4NO_3$ pour réaliser un échange des cations $Na^+$ de la zéolithe par des cations $NH_4{}^+$, les conditions opératoires de l'opération d'échange étant les suivantes :

| | |
|---|---|
| .volume de liquide par g de solide | 25 ml |
| .température de l'échange | 80 °C |
| .durée de l'échange | 1,5 heure |

L'opération d'échange était répétée cinq fois.

La forme acide de la zéolithe était ensuite obtenue par dégradation thermique des cations ammonium présents dans la zéolithe soumise à l'échange, ladite dégradation thermique étant réalisée sous un léger débit d'air de 1,5 l/heure selon le profil thermique ci-après :

| rampe de température | | durée (mn) |
|---|---|---|
| 20 °C | 200 °C | 120 |
| 200 °C | 200 °C | 60 |
| 200 °C | 550 °C | 240 |
| 550 °C | 550 °C | 180 |
| 550 °C | 25 °C | (inertie du four) |

La zéolithe acide résultant du traitement thermique ci-dessus était alors placée à l'humidificateur sous une humidité relative de 80% de façon à stabiliser son taux d'hydratation.

La zéolithe acide obtenue avait une structure de symétrie cubique et un rapport Si:Al égal à environ 4 et présentait un diagramme de diffraction des rayons X comparable à celui du tableau I, le paramètre $a_o$ de la maille cubique étant égal à 2,46nm. Le taux de fraction cristalline de cette faujasite acide, déterminé par diffraction des rayons X, était égal à 90%.

La composition chimique pondérale en oxydes de la zéolithe acide réhydratée comme indiqué ci-dessus est la suivante :

| $Na_2O$ | $SiO_2$ | $Al_2O_3$ | $H_2O$ |
|---|---|---|---|
| 0,44% | 61,5% | 12,9% | 25,0% |

La formule trouvée pour cette zéolithe, ramenée à une maille de la structure cubique, s'écrit à l'état anhydre (t = o dans la formule générale) :

$(2,1\ Na^+)\ (35,9\ H^+)\ [(SiO_2)_{154,0}\ (AlO_2)_{38,0}]^{38,0-}$

EXEMPLE 2 :

Etude de l'acidité par thermodésorption programmée d'ammoniac

En faisant appel à la technique de thermodésorption programmée d'ammoniac, on étudiait la répartition des sites acides des trois zéolithes acides suivantes :

A) - zéolithe acide selon l'invention obtenue comme décrit dans l'exemple 1 ;

B) - zéolithe acide de type faujasite (symétrie cubique) obtenue en soumettant une zéolithe Y dans sa forme ammonium $NH_4Y$, qui est commercialisée sous l'appellation ZF 110 par la société ZEOCAT, à un traitement de dégradation thermique des cations $NH_4^+$ identique à celui décrit dans l'exemple 1. Cette zéolithe acide, après réhydratation sous une humidité relative de 80% avait les caractéristiques physico-chimiques données dans le tableau II ci-après.

TABLEAU II

| Composition chimique pondérale en oxydes (%) | | | | Rapport Si:Al mesuré | Fraction cristalline*) |
|---|---|---|---|---|---|
| $Na_2O$ | $SiO_2$ | $Al_2O_3$ | $H_2O$ | | |
| 1,14 | 53,44 | 17,62 | 27,80 | 26 | 90 |

*) La référence utilisée est la zéolithe acide de l'exemple 1.

La formule trouvée pour la zéolithe ZF 110 dans sa forme acide, ramenée à une maille de sa structure cubique, s'écrit à l'état anhydre

$(5{,}7\ Na^+)\ (47{,}9\ H^+)\ [(SiO_2)_{138{,}4}\ (AlO_2)_{53{,}6}]^{53{,}6-}$

C) - zéolithe acide de type faujasite (symétrie cubique) consistant en la forme acide d'une zéolithe Y désaluminée commerciale (zéolithe ZF 510 de la société ZEOCAT) sans débris aluminique.

Les caractéristiques physico-chimiques de l'échantillon de cette zéolithe acide après réhydratation sous une humidité relative de 80% sont données dans le tableau III.

TABLEAU III

| Composition chimique pondérale en oxydes (%) | | | | Rapport Si:Al mesuré | Fraction cristalline*) |
|---|---|---|---|---|---|
| $Na_2O$ | $SiO_2$ | $Al_2O_3$ | $H_2O$ | | |
| 0,2 | 66,6 | 6,64 | 26,6 | 8,5 | 95 |

*) La référence utilisée est la zéolithe acide de l'exemple 1.

La formule trouvée pour la zéolithe acide ZF 510, ramenée à une maille de sa structure cubique, s'écrit à l'état anhydre

$(1{,}0\ Na^+)\ (19{,}2\ H^+)\ [(SiO_2)_{171{,}8}\ (AlO_2)_{20{,}2}]^{20{,}2-}$

Les opérations successives effectuées au cours de chaque essai de thermodésorption étaient réalisées sous un léger débit de gaz, ces opérations étant schématisées dans le tableau IV

TABLEAU IV

| Opération | Température (°C) | Durée (mn) | Gaz | |
|---|---|---|---|---|
| | | | Nature | Débit (l/h) |
| Activation de la zéolithe en deux stades | 200<br>550 | 30<br>60 | Hélium | 1,5 |
| Adsorption de $NH_3$ | 100 | 15 | $NH_3$ | 1,5 |
| Purge de l'excès de $NH_3$ | 140 | 30 | $NH_3$ | 4,0 |
| Désorption de $NH_3$ (un palier de désorption de 60 minutes est réalisé à chaque température) | 200<br>250<br>300<br>350<br>400<br>450<br>500<br>550 | 60<br>60<br>60<br>60<br>60<br>60<br>60<br>60 | Hélium | 1,5 |

A partir du volume de $NH_3$ libéré pour chaque palier de désorption, on déterminait la quantité correspondante de cations $H^+$ et rapportait cette quantité en milliéquivalents par gramme (meq $H^+$/g) de la zéolithe acide soumise à l'essai de thermodésorption.
Les résultats obtenus sont rassemblés dans le tableau V.

TABLEAU V

| Température (°C) | Quantité de $H^+$ dans la zéolithe acide(meq $H^+$/g) | | |
|---|---|---|---|
| | Zéolithe acide A | Zéolithe acide B | Zéolithe acide C |
| 150 | 0,020 | 0,040 | 0,029 |
| 200 | 0,392 | 0,297 | 0,226 |
| 250 | 0,372 | 0,257 | 0,215 |
| 300 | 0,342 | 0,229 | 0,213 |
| 350 | 0,367 | 0,197 | 0,214 |
| 400 | 0,271 | 0,142 | 0,124 |
| 450 | 0,119 | 0,086 | 0,044 |
| 500 | 0,057 | 0,052 | 0,026 |
| 550 | 0,045 | 0,043 | 0,029 |
| Acidité totale | 1,985 | 1,343 | 1,120 |

Au vu des résultats présentés dans le tableau V, il apparaît que la densité de sites acides et le nombre de sites de fortes acidités sont plus importants pour la zéolithe acide A (selon l'invention) que pour les zéolithes acides témoins B et C.

EXEMPLE 3:

Activité catalytique de la zéolithe acide en craquage du n-heptane

On évaluait les activités catalytiques de la zéolithe acide A selon l'invention et de chacune des zéolithes acides témoins B et C de l'exemple 2 en craquage catalytique du n-heptane.
Pour ce faire, on opérait dans un réacteur en quartz ayant un diamètre intérieur de 18mm et équipé en son centre d'une plaque de verre fritté sur laquelle on plaçait 500mg de la zéolithe acide à soumettre à l'essai. Après activation de la zéolithe placée sur la plaque de verre par chauffage à 200°C pendant 30mn puis à

550 °C pendant 60mn sous un courant d'azote ayant un débit de 1,5 l/heure, on envoyait sur la zéolithe activée un mélange d'azote et de n-heptane.

Les conditions opératoires étaient les suivantes :

. pression : atmosphérique
. rapport des pressions partielles
  $p_{N2} : P_{n-heptane}$ : 6,6
. température de la réaction : 350 °C
. (poids de réactif/poids de zéolithe)
  par heure : 2,4 $h^{-1}$

On donne dans le tableau VI la valeur de l'activité catalytique, exprimée en millimoles de n-heptane craqué par gramme de zéolithe et par heure (mmoles.$h^{-1}$.$g^{-1}$), en fonction du temps de réaction de la zéolithe en minutes (mn).

TABLEAU VI

| Temps de réaction (mn) | n-heptane craqué (mmoles.$h^{-1}$.$g^{-1}$) | | |
|---|---|---|---|
| | Zéolithe acide A | Zéolithe acide B | Zéolithe acide C |
| 8 | 14,67 | 3,61 | 13,88 |
| 18 | 10,66 | 2,69 | 8,31 |
| 28 | 8,20 | 2,25 | 5,67 |
| 38 | 6,95 | 1,88 | 4,72 |
| 48 | 6,01 | 1,55 | 3,90 |
| 68 | 4,53 | 1,29 | 2,47 |
| 125 | 2,26 | 0,64 | 1,17 |

Les résultats ci-dessus font ressortir que la zéolithe acide A selon l'invention présente une activité catalytique supérieure à celle des zéolithes acides témoins B et C.

EXEMPLE 4 :

Stabilité thermique de la zéolithe acide

Des échantillons de la zéolithe acide A (selon l'invention) et des zéolithes acides témoins B et C de l'exemple 2 étaient calcinés à 800 °C pendant 4 heures sous un débit d'air sec de 1,5 l/heure.

Les produits calcinés obtenus étaient réhydratés sous une humidité relative de 80%, après quoi on déterminait leur taux de fraction cristalline par diffraction des rayons X en prenant comme référence la zéolithe acide A (zéolithe acide de l'exemple 1).

Les taux de fraction cristalline des zéolithes A, B et C calcinées comme indiqué ci-dessus étaient respectivement de 90% (A calcinée), 25% (B calcinée) et 110% (C calcinée).

EXEMPLE 5 :

Influence de la stabilité thermique de la zéolithe acide sur ses caractéristiques catalytiques :

Les zéolithes acides A et C calcinées de l'exemple 4, qui ont une cristallinité comparable, étaient soumises à l'essai de thermodésorption programmée de $NH_3$ comme indiqué dans l'exemple 2 et également à l'essai de craquage catalytique du n-heptane comme indiqué dans l'exemple 3.

Les résultats de ces essais sont présentés respectivement dans les tableaux VII et VIII.

TABLEAU VII

| Thermodésorption programmée de $NH_3$ | | |
|---|---|---|
| Température (°C) | Acidité (meq $H^+$/g) | |
| | Zéolithe A calcinée de l'exemple 4 | Zéolithe C calcinée de l'exemple 4 |
| 150 | 0,024 | 0,032 |
| 200 | 0,220 | 0,180 |
| 250 | 0,175 | 0,130 |
| 300 | 0,129 | 0,099 |
| 350 | 0,087 | 0,076 |
| 400 | 0,049 | 0,045 |
| 450 | 0,026 | 0,019 |
| 500 | 0,021 | 0,014 |
| 550 | 0,019 | 0,016 |
| Acidité totale | 0,750 | 0,611 |

TABLEAU VIII

| Craquage du n-heptane | | |
|---|---|---|
| Temps de réaction (mn) | n-heptane craqué ($mmoles.h^{-1}.g^{-1}$) | |
| | Zéolithe A calcinée de l'exemple 4 | Zéolithe C calcinée de l'exemple 4 |
| 8 | 7,34 | 3,8 |
| 18 | 1,27 | 2,13 |
| 28 | 0,64 | 1,62 |
| 38 | 0,74 | 1,22 |
| 48 | 0,64 | 0,99 |
| 68 | 0,63 | 0,51 |
| 125 | 0,60 | 0,19 |

La zéolithe acide selon l'invention (zéolithe A) possède encore, après calcination à 800°C sous air, des caractéristiques catalytiques supérieures à celles de la zéolithe témoin C calcinée dans les mêmes conditions.

EXEMPLE 6 :

Stabilité hydrothermique de la zéolithe acide

On déterminait la surface spécifique d'échantillons des zéolithes acides A, B et C de l'exemple 2 et d'une zéolithe acide US Y (zéolithe acide Y ultra-stabilisée) provenant de la société KATALISTIKS (zéolithe acide D), avant et après avoir soumis lesdits échantillons à l'action de 100% de vapeur d'eau à 750°C pendant 2 heures.
La détermination des surfaces spécifiques était effectuée par adsorption d'azote selon la méthode B.E.T. simplifiée à un point.
Les résultats obtenus sont rassemblés dans le tableau IX.

TABLEAU IX

| Zéolithe acide utilisée | Surface spécifique ($m^2/g$) | | Rétention de surface (%) |
|---|---|---|---|
| | Avant traitement à la vapeur d'eau | Après traitement à la vapeur d'eau | |
| A | 684 | 414 | 61 |
| B | 585 | 9 | 1,5 |
| C | 615 | 542 | 88 |
| D | 562 | 432 | 77 |

Le rapport Si:Al de réseau de la zéolithe acide D est d'environ 4.

EXEMPLE 7:

Essai de laboratoire d'évaluation de l'activité catalytique sur charge réelle ("Microactivity test") :

On déterminait l'efficacité de deux catalyseurs A (selon l'invention) et D (comparatif) comme catalyseurs de craquage en lit fluidisé (FCC) en utilisant la méthode de laboratoire ("Microactivity test") définie dans la norme ASTM D 3907-87. La charge hydrocarbonée traitée était une charge réelle prélevée en raffinerie, dont les caractéristiques sont résumées ci-après :

. $d_4^{15}$ = 0,922
. point d'aniline = 82,1°C
. viscosité à 100°C = $6{,}08.10^{-6}$ $m^2/s$
. teneur en soufre = 2,68% en poids
. teneur en composés azotés = 800 ppm (exprimés en azote)
. carbone Conradson = 0,44%
. distillation selon ASTM D 1160
5 % 324°C
10 % 362°C
50 % 431°C
90 % 502°C
95 % 519°C

Les catalyseurs A et D utilisés étaient les suivants :

. Catalyseur A (selon l'invention): obtenu à partir d'un mélange renfermant, en poids, 95% d'un catalyseur E et 5% de la zéolithe acide A de l'exemple 3 (zéolithe acide de l'exemple 1). Le catalyseur E consistait en un catalyseur industriel de craquage catalytique de la société CROSFIELD (catalyseur SLS 100) soumis à un traitement à la vapeur d'eau à 750°C pendant 17 heures sous 100% de vapeur d'eau.
. Catalyseur D (comparatif) : obtenu à partir d'un mélange renfermant, en poids, 95% du catalyseur E précité et 5% de la zéolithe acide D.

Les conditions opératoires utilisées dans l'essai étaient telles qu'indiquées ci-après :

. quantité de catalyseur : 3g
. température du réacteur : 530°C
. rapport pondéral catalyseur : charge : 6
. (poids de charge/poids de catalyseur)/h : (30g/g)/heure

Les résultats obtenus sont résumés dans le tableau X.

Ces résultats font apparaître que le catalyseur A possède une activité équivalente à celle du catalyseur D. Par ailleurs, le catalyseur A présente une meilleure sélectivité en coke et en essence, les GPL obtenus étant plus riches en oléfines, ce qui laisse présager un indice d'octane plus élevé pour les essences obtenues.

TABLEAU X

| | Catalyseur A | Catalyseur D |
|---|---|---|
| Rendements (% poids) | 7,74 | 8,55 |
| Coke | 0,09 | 0,09 |
| $H_2$ | 1,25 | 1,32 |
| $C_1$ | 2,33 | 2,42 |
| Gaz ($H_2 + C_1 + C_2$) | 3,67 | 3,83 |
| $C_3$ | 3,69 | 4,43 |
| $C3^=$ | 4,43 | 4,16 |
| iso $C_4$ | 7,05 | 7,18 |
| n-$C_4$ | 2,33 | 2,55 |
| $C_4^=$ | 4,34 | 4,38 |
| GPL ($\Sigma C_3 + \Sigma C_4$) | 21,84 | 22,70 |
| Total gaz | 25,51 | 26,53 |
| Essence totale (Point final 220 °C) | 47,01 | 45,28 |
| Conversion (% poids) (Essence + Gaz + Coke) | 80,26 | 80,36 |
| Gazole (220 °C-350 °C) | 13,45 | 13,45 |
| Résidus (>350 °C) | 6,29 | 6,19 |
| Rapport $C_3^=:\Sigma C_3$ | 0,55 | 0,48 |
| Rapport $C_4^=:\Sigma C_4$ | 0,32 | 0,31 |

**Revendications**

**1.** Catalyseur comportant, en poids, y% d'un liant inorganique et z% d'une zéolithe protonée à structure faujasite de rapport Si:Al de synthèse au moins égal à 3, y et z étant des nombres tels que $1 \leqq y \leqq 99$ et $1 \leqq z \leqq 99$ avec $y + z = 100$, ladite zéolithe présentant un paramètre $a_o$ de sa maille cubique tel que $2{,}42nm \leqq a_o \leqq 2{,}48nm$ et répondant à une formule qui, ramenée à une maille cubique, s'écrit:

$$(uM_1q+)\ (vH+)\ [(SiO_2)_{192-x}\ (AlO_2)_x]^{x-}\ (t\ H_2O)$$

avec dans cette formule, x désignant un nombre tel que $6 \leqq x \leqq 48$, $M_1q+$ désignant des cations d'au moins un métal de valence q choisi parmi les métaux alcalins et les métaux alcalinoterreux, et u, v, x et t représentant des nombres tels que $t \geqq O$ selon le degré d'hydratation de la zéolithe, t=O pour une zéolithe complètement anhydre, et que $qu + v \geqq x$, ledit catalyseur se caractérisant en ce que le nombre v de cation H+ dans la formule de la zéolithe est tel que $0{,}5\ x \leqq v \leqq x$ et en ce que ladite zéolithe protonée est issue d'une faujasite alcaline de rapport Si:Al de synthèse au moins égal à 3 ou d'une telle faujasite désaluminée, laquelle faujasite alcaline est obtenue en formant un gel d'aluminosilicate à partir d'un mélange réactionnel, qui a un pH supérieur à 10 et renferme de l'eau, une source de silicium tétravalent, une source d'aluminium trivalent, une source d'ions hydroxydes sous la forme d'hydroxyde de métal alcalin ou alcalinoterreux et un agent structurant ST consistant en au moins un compose choisi dans le groupe formé (i) des macrocycles et macropolycycles comportant dans le cycle ou les cycles des hétéroatomes pris parmi O, N, Si et S et renfermant 10 à 17 atomes dans le cycle pour les macrocycles et 10 à 18 atomes dans chaque cycle pour les macropolycycles et (ii) des composés acycliques oxygénés de formule

$$R_1O \left[ C_mH_{2m-1}\ Y - O \right]_g R_2\ ,$$

dans laquelle $R_1$ et $R_2$, identiques ou différents, représentent chacun un atome d'hydrogène ou un radical alcoyle en $C_1$ à $C_4$, Y désigne un atome d'hydrogène ou un radical -OH, m est égal à 2 ou 3 et peut être différent d'un motif à l'autre et g est un nombre allant de 1 à 12, ledit gel d'aluminosilicate

présentant, en termes de proportions molaires, une composition telle que l'on ait 1 $Al^{III}$ : 2 à 20 $Si^{IV}$ : 0,5 à 8 $OH^-$ : 0,1 à 4 ST : 40 à 200 $H_2O$, puis en maintenant le gel obtenu à une température au plus égale à 150 °C et sous une pression au moins égale à la pression autogène du mélange constitué par ledit gel pendant une durée suffisante pour effectuer la cristallisation de ce gel en un précurseur de la faujasite alcaline consistant en ladite faujasite emprisonnant l'agent structurant ST dans ses cavités et en soumettant ledit précurseur à une calcination pour détruire l'agent structurant et produire la faujasite alcaline.

**2.** Catalyseur selon la revendication 1, caractérisé en ce que les proportions y% du liant inorganique et z% de la zéolithe sont telles que $4 \leqq y \leqq 96$ et $4 \leqq z \leqq 96$ avec $y+z=100$.

**3.** Catalyseur selon la revendication 1 ou 2, caractérisé en ce que la zéolithe présente un rapport Si:Al allant de 3 à 20.

**4.** Catalyseur selon l'une des revendications 1 à 3, caractérisé en ce que le nombre v de cations H+ dans la formule de la zéolithe est tel que $0,8\,x \leqq v \leqq x$.

**5.** Catalyseur selon l'une des revendications 1 à 4, caractérisé en ce que dans la formule de la zéolithe $M_1q+$ représente des cations d'au moins un élément choisi parmi Na, K, Li, Cs, Ca, Sr et Ba.

**6.** Catalyseur selon l'une des revendications 1 à 5, caractérisé en ce que la zéolithe présente dans le catalyseur possède une surface spécifique, déterminée par adsorption d'azote selon la méthode B.E.T. simplifiée à un point (norme NF X 11-622), comprise entre $100m^2/g$ et $1000m^2/g$ et de préférence entre $300m^2/g$ et $900m^2/g$.

**7.** Catalyseur selon l'une des revendications 1 à 6, caractérisé en ce qu'il renferme en plus du liant et de la zéolithe jusqu'à 15% et de préférence jusqu'à 10%, en poids de l'ensemble liant et zéolithe, d'au moins un métal catalytiquement actif, présent à l'état élémentaire ou sous la forme d'un composé métallique et choisi parmi les métaux nobles tels que platine, palladium, rhodium et iridium, les métaux de transition non nobles tels que Ni, Co, W et Mo et certains autres métaux tels que gallium.

**8.** Catalyseur selon l'une des revendications 1 à 7, caractérisé en ce que la zéolithe présente, après calcination à 600 °C pendant 4 heures, un diagramme de diffraction des rayons X comparable à celui du tableau I donné dans la description.

**9.** Catalyseur selon l'une des revendications 1 à 8, caractérisé en ce que le liant inorganique consiste en un ou plusieurs oxydes réfractaires choisis parmi alumine, silice, argile, silice/alumine et magnésie.

**10.** Catalyseur selon l'une des revendications 1 à 9, caractérisé en ce que le gel d'aluminosilicate, à partir duquel on cristallise la faujasite alcaline, présente, en termes de proportions molaires, une composition telle que l'on ait 1 $Al^{III}$ : 4 à 10 $Si^{IV}$ : 1 à 6 $OH^-$ : 0,1 à 1 ST : 50 à 150 $H_2O$.

**11.** Application du catalyseur selon l'une des revendications 1 à 10, comme promoteur des réactions de catalyse acide réalisées sur des charges hydrocarbonées.

**12.** Application selon la revendication 11, caractérisée en ce que la réaction de catalyse acide est un craquage catalytique, notamment un craquage catalytique de distillats sous vide ou de résidus en essence, en opérant en particulier en lit fluidisé ou en lit mobile à des températures allant, par exemple, de 450 °C à 650 °C.

**13.** Application selon la revendication 11, caractérisée en ce que la réaction de catalyse acide est un hydrocraquage catalytique, notamment un hydrocraquage de distillats sous vide en gazole, en opérant à des températures supérieures à 250 °C, en particulier entre 350 °C et 500 °C, sous une pression supérieure à 15 bars, avantageusement supérieure à 30 bars, et avec une vitesse volumique horaire comprise entre 0,2 et 10.

**14.** Application selon la revendication 11, caractérisée en ce que la réaction de catalyse acide est une réaction d'isomérisation, en particulier isomérisation des hydrocarbures alcoylaromatiques tels que les

xylènes.

**15.** Application selon la revendication 11, caractérisée en ce que la réaction de catalyse acide est une alkylation d'hydrocarbures aromatiques, notamment benzène, par les oléfines.

## Claims

**1.** Catalyst comprising, by weight, y% of an inorganic binder and z% of a protonised zeolite with a faujasite structure with an Si:Al ratio from synthesis which is at least equal to 3, y and z being numbers such that $1 \leqq y \leqq 99$ and $1 \leqq z \leqq 99$ where $y + z = 100$, said zeolite having a parameter $a_o$ of its cubic mesh such that $2.42 \text{ nm} \leqq a_o \leqq 2.48 \text{ nm}$ and corresponding to a formula which, applied to a cubic mesh, is written:

$$(uM_1 q+)\ (vH^+)\ [(SiO_2)_{192-x}\ (AlO_2)_x]^{x-}\ (t\ H_2O)$$

where, in this formula, x designates a number such that $6 \leqq x \leqq 48$, $M_1{}^{q+}$ designating cations of at least one metal of valence q selected from among the alkaline metals and the alkaline earth metals, and u, v, x and t representing numbers such that $t \geqq 0$ depending on the degree of hydration of the zeolite, $t = 0$ in the case of a completely anhydrous zeolite, and that $qu + v \geqq x$, said catalyst being characterised in that the number v of the cation $H^+$ in the formula of the zeolite is such that $0.5\ x \leqq v \leqq x$ and in that said protonised zeolite is produced from an alkaline faujasite with an Si:Al ratio from synthesis at least equal to 3 or from a dealuminised faujasite of this type, which alkaline faujasite is obtained by forming an aluminosilicate gel from a reactive mixture, which has a pH greater than 10 and contains water, a source of tetravalent silicon, a source of trivalent aluminium, a source of hydroxide ions in the form of hydroxide of an alkaline metal or alkaline earth metal and a structuring agent ST consisting of at least one compound selected in the group formed of (i) macrocycles and macropolycycles comprising, in the cycle or the cycles, heteroatoms taken from among O, N, Si and S and containing 10 to 17 atoms in the cycle in the case of macrocycles, and from 10 to 18 atoms in each cycle in the case of macropolycycles, and of (ii) oxygenated acyclic compounds of the formula:

$$R_1O\ \{C_mH_{2m-1}\ Y - O\}_g\ R_2,$$

in which $R_1$ and $R_2$ which are identical or different, each represent a hydrogen atom or a $C_1$ to $C_4$ alkyl radical, Y designates a hydrogen atom or an -OH radical, m is 2 or 3 and may be different from one motif to another, and g is a number from 1 to 12, said aluminosilicate gel having, in terms of molar proportions, a composition such that 1 $Al^{III}$ : 2 to 20 $Si^{IV}$ : 0.5 to 8 $OH^-$ : 0.1 to 4 ST : 40 to 200 $H_2O$, is obtained, whilst maintaining the gel obtained at a temperature of at most 150°C and under a pressure which is at least equal to the autogenous pressure of the mixture constituted by said gel for a duration which is sufficient to perform the crystallisation of this gel into a precursor of the alkaline faujasite consisting of said faujasite trapping the structuring agent ST in the cavities thereof and in subjecting said precursor to a calcination to destroy said structuring agent and produce the alkaline faujasite.

**2.** Catalyst according to Claim 1, characterised in that the proportions y% of inorganic binder and z% of zeolite are such that $4 \leqq y \leqq 96$ and $4 \leqq z \leqq 96$, where $y + z = 100$.

**3.** Catalyst according to Claim 1 or Claim 2, characterised in that the zeolite has an Si:Al ratio of from 3 to 20.

**4.** Catalyst according to any one of Claims 1 to 3, characterised in that the number v of cations $H^+$ in the formula of the zeolite is such that $0.8\ x \leqq v \leqq x$.

**5.** Catalyst according to any one of Claims 1 to 4, characterised in that in the formula of the zeolite $M_1{}^{q+}$ represents cations of at least one element selected from among Na, K, Li, Cs, Ca, Sr and Ba.

**6.** Catalyst according to any one of Claims 1 to 5, characterised in that the zeolite present in the catalyst has a specific surface determined by adsorption of nitrogen according to the BET method simplified to one point (standard NF X 11-622), of between 100 $m^2/g$ and 1000 $m^2/g$ and preferably of between 300 $m^2/g$ and 900 $m^2/g$.

7. Catalyst according to any one of Claims 1 to 6, characterised in that it contains, in addition to binder and zeolite up to 15%, and preferably up to 10%, by weight of the binder and zeolite together, of at least one catalytically active metal, which is present in the elementary state or in the form of a metallic compound and which is selected from among precious metals such as platinum, palladium, rhodium and iridium, the non-precious transition metals such as Ni, Co, W and Mo and certain other metals such as gallium.

8. Catalyst according to any one of Claims 1 to 7, characterised in that the zeolite has, after calcination at 600 °C for 4 hours, an X-ray diffraction diagram comparable to that in Table I given in the description.

9. Catalyst according to any one of Claims 1 to 8, characterised in that the inorganic binder consists of one or several heat-resistant oxides selected from among alumina, silica, clay, silica/alumina and magnesia.

10. Catalyst according to any one of Claims 1 to 9, characterised in that the aluminosilicate gel, from which the alkaline faujasite is crystallised, has, in terms of molar proportions, a composition such that 1 $Al^{III}$ : 4 to 10 $Si^{IV}$ : 1 to 6 $OH^-$ : 0.1 to 1 ST : 50 to 150 $H_2O$ is obtained.

11. Use of the catalyst according to any one of Claims 1 to 10 as a promoter of reactions of acid catalysis performed on hydrocarbon charges.

12. Use according to Claim 11, characterised in that the acid catalysis reaction is a catalytic cracking, in particular a catalytic cracking of vacuum distillates or of residues to form petrol, operating in particular on a fluid bed or a fluidized bed at temperatures of, for example, from 450 °C to 650 °C.

13. Use according to Claim 11, characterised in that the acid catalysis reaction is a catalytic hydrocracking, in particular a hydrocracking of vacuum distillates in gas oil, operating at temperatures of greater than 250 °C, in particular of between 350 °C and 500 °C, at a pressure of greater than 15 bar, and advantageously greater than 30 bar, and with horary volumetric speed of between 0.2 and 10.

14. Use according to Claim 11, characterised in that the acid catalysis reaction is an isomerisation reaction, in particular isomerisation of aromatic alkyl hydrocarbons such as xylenes.

15. Use according to Claim 11, characterised in that the acid catalysis reaction is an alkylation of aromatic hydrocarbons, in particular benzene, by the olefins.

**Patentansprüche**

1. Katalysator, der, bezogen auf das Gewicht, y % eines anorganischen Bindemittels und z % eines protonenhaltigen Zeolithen mit Faujasitstruktur bei einem Si:Al-Syntheseverhältnis von wenigstens 3 enthält, wobei y und z den Zahlenwerten $1 \leqq y < 99$ und $1 \leqq z \leqq 99$ bei $y + z = 100$ entsprechen, und der Parameter $a_o$ der kubischen Elementarzelle des Zeolithen die Werte 2,42 nm $\leqq a_o \leqq$ 2,48 nm aufweist und dieser einer Formel entspricht die sich, zurückgeführt auf eine kubische Elementarzelle, mit

$$(uM_1{}^{q+})(vH^+)[(SiO_2)_{192-x}(AlO_2)_x]^{x-}\ (t\ H_2O)$$

beschreiben läßt, wobei in dieser Formel x einem Zahlenwert von $6 \leqq x \leqq 48$ entspricht, $M_1{}^{q+}$ Kationen wenigstens eines Metalls mit der Wertigkeit q, ausgewählt unter Alkali- und Erdalkalimetallen, bedeutet, und u, v, x und t den Zahlenwerten $t \geqq 0$ je nach dem Hydratisierungsgrad des Zeolithen, $t = 0$ bei einem vollständig wasserfreien Zeolithen, und $qu + v \geqq x$ entsprechen, dadurch gekennzeichnet, daß die Zahl v der Kationen $H^+$ in der Formel des Zeolithen einem Wert $0,5\ x \leqq v \leqq x$ entspricht und der protonenhaltige Zeolith erhalten wird aus einem alkalischen Faujasit mit einem Si:Al-Syntheseverhältnis von wenigstens gleich 3 oder einem solchen entaluminierten Faujasit, wobei man den alkalischen Faujasit erhält, indem man ein Alumosilikatgel ausgehend von einem Reaktionsgemisch bildet, das einen pH von über 10 aufweist und Wasser, eine Quelle von vierwertigem Silizium, eine Quelle von dreiwertigem Aluminium, eine Hydroxidionenquelle in Form eines Alkali- oder Erdalkalimetallhydroxids und ein Strukturierungsmittel ST enthält, das wenigstens aus einer Verbindung besteht, ausgewählt aus

der Gruppe, gebildet (i) aus Macrocyclen und Macropolycyclen, die im Cyclus bzw. in den Cyclen unter O, N, Si und S ausgewählte Heteroatome umfassen und im Falle der Macrocyclen 10 bis 17 Atome im Cyclus und im Falle der Macropolycyclen 10 bis 18 Atome in jedem Cyclus enthalten, und (ii) acyclischen Sauerstoffverbindungen der Formel

$$R_1O \left[ C_mH_{2m-1}\ Y - O \right]_g R_2,$$

worin $R_1$ und $R_2$ gleiche oder unterschiedliche Bedeutungen haben und jeweils ein Wasserstoffatom oder einen $C_1$-$C_4$-Alkylrest darstellen, Y ein Wasserstoffatom oder einen -OH-Rest bezeichnet, m 2 oder 3 bedeutet und von einer zur anderen Gruppe unterschiedlich sein kann und g eine Zahl von 1 bis 12 bedeutet, das Alumosilikatgel, ausgedrückt in Molarverhältnissen, eine Zusammensetzung von 1 $Al^{III}$ : 2 bis 20 $Si^{IV}$ : 0,5 bis 8 $OH^-$ : 0,1 bis 4 ST : 40 bis 200 $H_2O$ aufweist, wonach man das erhaltene Gel bei einer Temperatur von höchstens 150 °C und unter einem Druck, der zumindest dem Eigendruck des vom Gel gebildeten Gemisches entspricht, während einer Zeitdauer hält, die ausreicht, um die Kristallisation dieses Gels zu einem Precursor des alkalischen Faujasits zu bewirken. der aus dem Faujasit besteht, der in seinen Hohlräumen das Strukturierungsmittel ST umschließt, und den Precursor zur Zerstörung des Strukturierungsmittels und Erzeugung des alkalischen Faujasits glüht.

**2.** Katalysator nach Anspruch 1, dadurch **gekennzeichnet**, daß die Mengen y % des anorganischen Bindemittels dem Wert $4 \leqq y \leqq 96$ und die Mengen z % des Zeolithen dem Wert $4 \leqq z \leqq 96$ bei $y + z = 100$ entsprechen.

**3.** Katalysator nach Anspruch 1 oder 2, dadurch **gekennzeichnet**, daß der Zeolith ein Si:Al-Verhältnis von 3 bis 20 aufweist.

**4.** Katalysator nach einem der Ansprüche 1 bis 3, dadurch **gekennzeichnet**, daß die Zahl v der Kationen $H^+$ in der Formel des Zeolithen dem Wert $0{,}8\,x \leqq v \leqq x$ entspricht.

**5.** Katalysator nach einem der Ansprüche 1 bis 4, dadurch **gekennzeichnet**, daß in der Formel des Zeolithen $M_1{}^{q+}$ Kationen wenigstens eines Elements darstellt, ausgewählt unter Na, K, Li, Cs, Ca, Sr und Ba.

**6.** Katalysator nach einem der Ansprüche 1 bis 5, dadurch **gekennzeichnet**, daß der im Katalysator vorliegende Zeolith eine spezifische Oberfläche, ermittelt durch Stickstoffadsorption nach der vereinfachten BET-Einpunktmethode (Norm NF X 11-622), zwischen 100 $m^2/g$ und 1000 $m^2/g$ und vorzugsweise zwischen 300 $m^2/g$ und 900 $m^2/g$ besitzt.

**7.** Katalysator nach einem der Ansprüche 1 bis 6, dadurch **gekennzeichnet**, daß er neben dem Bindemittel und dem Zeolithen bis zu 15 % und vorzugsweise bis zu 10 %, bezogen auf das Gesamtgewicht von Bindemittel und Zeolith, wenigstens eines katalytisch aktiven Metalls in elementarem Zustand oder in Form einer Metallverbindung enthält, und das Metall ausgewählt ist unter Edelmetallen wie Platin, Palladium, Rhodium und Iridium, nichtedlen Übergangsmetallen wie Ni, Co, W und Mo und einigen anderen Metallen wie Gallium.

**8.** Katalysator nach einem der Ansprüche 1 bis 7, dadurch **gekennzeichnet**, daß der Zeolith nach Glühen bei 600 °C während vier Stunden ein Röntgenbeugungsdiagramm aufweist. das mit dem in Tabelle I des Beschreibungstextes vergleichbar ist.

**9.** Katalysator nach einem der Ansprüche 1 bis 8, dadurch **gekennzeichnet**, daß das anorganische Bindemittel aus einem oder mehreren feuerfesten Oxiden besteht, ausgewählt unter Tonerde, Kieselerde, Ton, Kieselerde/Tonerde und Magnesia.

**10.** Katalysator nach einem der Ansprüche 1 bis 9, dadurch **gekennzeichnet**, daß das Alumosilikatgel, aus dem man den alkalischen Faujasit kristallisiert eine Zusammensetzung, ausgedrückt in Molarverhältnissen, von 1 $Al^{III}$ : 4 bis 10 $Si^{IV}$ : 1 bis 6 $OH^-$ : 0,1 bis 1 ST : 50 bis 150 $H_2O$ aufweist.

11. Verwendung des Katalysators nach einem der Ansprüche 1 bis 10 als Beschleuniger für an Kohlenwasserstofffraktionen durchgeführten Säurekatalysereaktionen.

12. Verwendung nach Anspruch 11, dadurch **gekennzeichnet**, daß die Säurekatalysereaktion ein Katkrackprozeß ist, insbesondere ein Katkracking von Destillaten im Vakuum oder von Rückständen zu Benzin, insbesondere in einer Wirbelschicht bzw. einem Fließbett, bei Temperaturen z.B. zwischen 450 und 650 ° C.

13. Verwendung nach Anspruch 11, dadurch **gekennzeichnet**, daß die Säurekatalysereaktion ein katalytisches Hydrokracking, insbesondere ein Hydrokracking von Destillaten im Vakuum zu Dieselkraftstoff bei Temperaturen von über 250 ° C, insbesondere zwischen 350 und 500 ° C, unter einem Druck von über 15 bar, vorteilhafterweise von über 30 bar, und bei einer Raumgeschwindigkeit pro Stunde von 0,2 bis 10 ist.

14. Verwendung nach Anspruch 11, dadurch **gekennzeichnet**, daß die Säurekatalysereaktion eine Reaktion der Isomerisierung, insbesondere von alkylaromatischen Kohlenwasserstoffen wie Xylolen ist.

15. Verwendung nach Anspruch 11, dadurch **gekennzeichnet**, daß die Säurekatalysereaktion eine Alkylierung von aromatischen Kohlenwasserstoffen, insbesondere von Benzol, mit Olefinen ist.